# EUROPEAN PATENT SPECIFICATION

(11) **EP 4 345 169 B1**
(45) Date of publication and mention of the grant of the patent: **31.07.2024**
(21) Application number: 23199266.0
(22) Date of filing: 25.09.2023
(51) Int. Cl.: C12Q 1/6841

(54) **GENE SPECIFIC TISSUE INFORMATION AND SEQUENCING**
GENSPEZIFISCHE GEWEBEINFORMATIONEN UND SEQUENZIERUNG
INFORMATIONS DE TISSU SPÉCIFIQUES DE GÈNES ET SÉQUENÇAGE

(30) Priority: 29.09.2022 EP 22198689
(43) Date of publication of application: 03.04.2024
(73) Proprietor: Miltenyi Biotec B.V. & Co. KG, 51429 Bergisch Gladbach (DE)
(72) Inventor: PINARD, Robert, 51429 Bergisch Gladbach (DE); HOSONO, Seiyu, 51429 Bergisch Gladbach (DE); MUELLER, Reto, 51429 Bergisch Gladbach (DE); NEIL, Emily, 51429 Bergisch Gladbach (DE)
(74) Representative: Kisters, Michael Marcus

(56) References cited:
- EP-A1- 3 936 623
- EP-A1- 4 039 822
- US-A1- 2022 042 083
- US-A1- 2022 186 300
- US-A1- 2022 235 403
- MICHAELA ASP ET AL: "Spatially Resolved Transcriptomes-Next Generation Tools for Tissue Exploration", BIOESSAYS, JOHN WILEY & SONS LTD, GB, vol. 42, no. 10, 4 May 2020 (2020-05-04), pages 1 - 16, XP071527643, ISSN: 0265-9247, DOI: 10.1002/BIES.201900221
- MOFFITT JEFFREY R ET AL: "The emerging landscape of spatial profiling technologies.", NATURE REVIEWS. GENETICS 12 2022, vol. 23, no. 12, 20 June 2022 (2022-06-20), pages 741 - 759, XP055968494, ISSN: 1471-0064

## Description

### BACKGROUND

It is challenging to detect all expressed genes on a sub cellular level on tissue and simultaneously obtain the sequence and/or spatial information of these genes.

This invention describes the method for recognizing and amplifying a specific region on a messenger RNA (mRNA) using a probe containing non pre-defined and randomly generated Unique Molecular Identifier (UMI) juxtaposed to a portion targeting a region of interest which contains a mutation or sequence variant on the mRNA captured by reverse transcription, and a gap padlock locator probe copying the sequence information of the linear probe UMI. The ligated padlock probe is used as a circular template to generate locator DNA nanoballs using Rolling Circle Amplified (RCA). Following the sequencing of the locator DNA nanoballs, the cDNA with UMI information is retrieved, extracted, amplified and sequenced by NGS.

### SUMMARY

Accordingly, it was an object of the invention to provide a method to simultaneously obtain both the spatial location and sequence information of a target sequence with a higher resolution than the known technologies. Document EP4039822 relates to spatial transcription determination based on gap-filling barcoded padlock probes having a spatial unique molecular identifier (SUMI) and which directly bind to the target, this document does not describe reverse transcription of linear nucleic acid probes. Document EP3936623 relates to gene expression mapping comprising the use of padlock probes comprising SUMI and which bind directly to the target, this document makes use of multiple detection probes and makes not mention of reverse transcribing linear probes.

Here we describe a method to
(1) Spatially localize the mRNA expressed on a tissue by the use of a hybrid circular linear DNA probe with UMI.
(2) The nucleotide information of an adj acent region downstream from the region where the linear portion of a probe with UMI is copied with reverse transcriptase. Concurrently a padlock locator probe whose ends are complementary to target sequences flanking the UMI portion on the linear portion of the probes are hybridized creating a small gap corresponding to the length of the UMI that can be copied using Phusion DNA polymerase generating a circular template .
(3) The circular template is used to generate DNA nanoballs for which the UMI portion are sequenced on the tissue, and used as reporters for the linear probes hybridization and mRNA coordinate generation.
(4) The UMI containing cDNA strand generate by reverse transcription is then extracted from the tissue.
(5) The extracted UMI containing cDNA strand is then amplified by PCR using primers with known branched adaptor ends.
(6) The PCR product is circularized and RCA amplified
(7) The RCA product can be sequenced by NGS.
(8) The sequenced obtained are matched to the actual tissue location using the UMI's

Object of the invention is a method to obtain the spatial location and sequence information of a target sequence of at least one m-RNA strand on a tissue sample comprising the steps
a. providing a linear probe, containing a) a binding region capable of binding to the at least one m-RNA strand and b) an anchor sequence comprising a UMI region located between a first and a second locator regions and c) a primer region;
b. hybridizing the linear probe with its binding region to the m-RNA strand;
c. complementing the linear probe using the m-RNA strand as template thereby obtaining a reversed transcribed c-DNA strand
d. hybridizing a locator molecule with its 3' and 5' ends to the first and second locator regions thereby creating a gap corresponding to the length of the UMI of the linear probe
e. Filling the gap in the locator molecule with nucleotides complementary to the UMI using a non-strand displacement enzyme thereby creating a circular template comprising a copy of the UMI region from the linear probe.
f. multiplying the circular template molecule by RCA on the tissue sample, starting from a primer region thereby creating a rolony
g. Sequencing at least the UMI portion of the rolonies thereby obtaining the spatial location of the m-RNA on the tissue
h. removing the reversed transcribed c-DNA strand from the tissue and dehybridizing the m-RNA strand thereby obtaining a single stranded c-DNA oligomer
i. providing the single stranded cDNA oligomer with a first and a second adaptor primer at the 3' and 5' ends obtaining a primed single stranded oligomer; amplification of the primed single stranded oligomer by PCR
j. Sequencing the amplified primed single stranded oligomer and linking the spatial information of the rolonies with the sequence information of the amplified primed single stranded oligomer via the UMI sequence

In a first embodiment, the locator molecule is hybridized first with its binding region to the m-RNA strand and then the linear probe is then hybridized to the locator molecule. In other words, steps a) to d) are performed in the sequence a), c), d), and d).

In a second embodiment, the locator molecule is hybridized first to the linear probe, which is then hybridized with its binding region to the m-RNA strand. In other words, steps a) to d) are performed in the sequence a), d), b), and c).

The present invention is directed to a method of performing a spatial localization of a gene of interest in a given tissue using a linear DNA primer containing a sequence complementary to a region upstream of a region of interest on an mRNA or a poly T sequence complementary to mRNA poly A tails and also contains a non-binding portion which includes a random sequence (UMI) located between two regions that are used for the hybridization of a padlock locator probe molecule (region A & B). The sequence information of a the particular region of interest on the mRNA is first captured by reverse transcription of the mRNA into a cDNA molecule using the linear DNA primer.

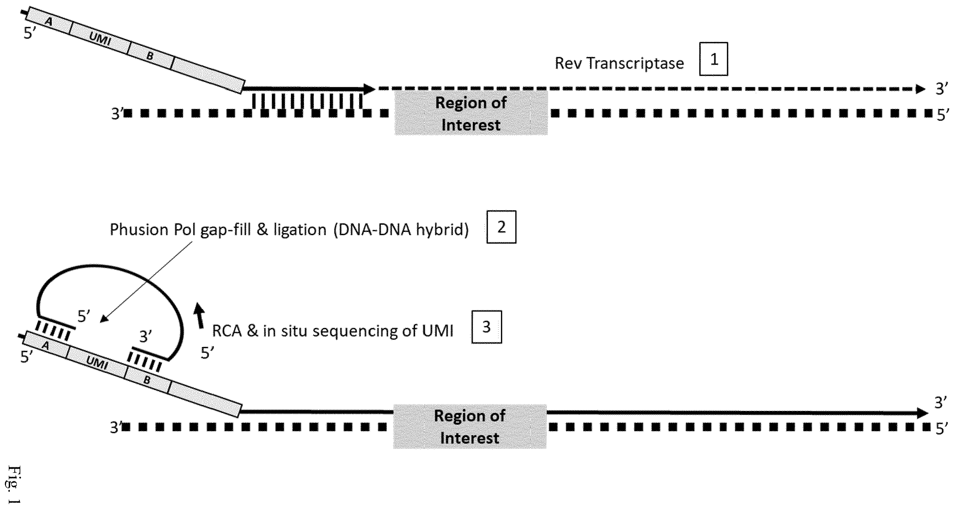

The padlock locator probe molecule can be either pre-attached to the non-binding region of linear DNA primer( regions A & B) or it can be hybridized to the linear DNA primer after the linear primer attaches to the sequence upstream of a desired region on an mRNA directly on a tissue (Fig 1).

The hybridization of the locator probe to the linear DNA primer creates an open circle padlock-like structure with a gap corresponding to the random sequence on the linear primer. The gap is filled by a non strand displacement enzyme and the two extremities are ligated forming a closed circular molecule that can then be amplified by rolling circle amplification (RCA) using an oligonucleotide primer directly on a tissue. The RCA generates DNA nanoballs (rolonies) at the same location where the mRNA was copied into a cDNA on the tissue section. The random and not pre-defined sequence can then be decoded by sequencing the RCA product on tissue where it is used a series of coordinates for each of the rolonies generated.

The cDNA generated with the linear primer is then physically retrieved and extracted from the tissue.

The retrieved cDNA is then PCR amplified using a PCR primer which anneals to the 5' and 3' end of the cDNA and carries DNA adapters.

The PCR amplified product can then be circularized using a splint bridge oligonucleotide primer which brings the two ends together.

The circle is then amplified by Rolling Circle Amplification (RCA). The RCA product is then sequenced.

By NGS sequencing the random sequence from the linear primer linked to the cDNA is identified and then can be assigned to the exact tissue location via the coordinates obtained by the sequencing of the same random sequence on the tissue.

By NGS sequencing the targeted region of interest on genomic DNA or mRNA, mutation or nucleotide variant can be analyzed.

### BRIEF DESCRIPTION OF THE DRAWINGS

Figure 1 shows the first three steps of the method of the invention
Figure 2 shows the circular molecule used as a template to generate DNA nanoballs.
Figure 3 shows how the extracted cDNA strand from the tissue can be PCR amplified and later circularized by adding adaptor regions (P1 and P2) to the PCR primers.

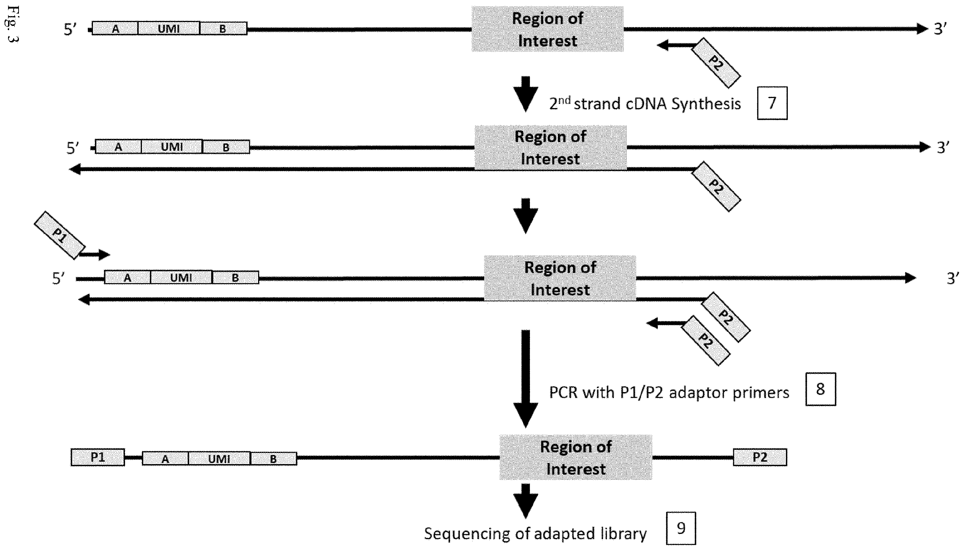

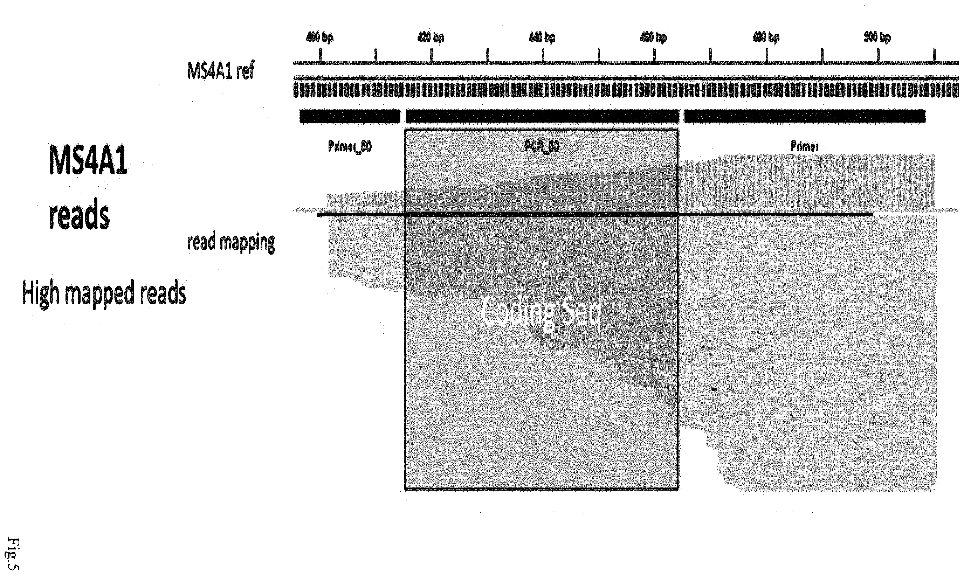

Figure 3 and 5 show experimental data

### DETAILED DESCRIPTION

The method of the invention is described in detail in Figs 1-3, and the term "steps" refers to the respective steps in the figures.

Spatial localization of mRNA molecule by RCA followed by ex-situ sequencing of the region of interest from a copied RNA molecule involves RCA and in situ sequencing of UMIs performed directly on tissue and consists of an eight step process. (Step 1) Hybridization of the linear probe containing a random sequence and two anchors (A & B) to mRNA on tissue via the specific sequence portion located at the 3'end and copying the downstream mRNA region of interest into a cDNA molecule by a reverse transcription using the linear probe as a primer. (Step 2) Hybridization of the circle locator padlock molecule to the linear probe via regions A & B, leaving a gap corresponding to the UMI region. Copying the UMI region by filling the gap using a non-strand displacement enzyme and ligation of the two extremities of the circle locator padlock using a DNA ligase, generating a circular molecule.

The circular molecule is used as a template to generate DNA nanoballs (rolonies) using RCA with a circle specific RCA primer (Step 3). The UMI portion of the rolonies are sequenced directly on tissue, providing the location coordinates of the target regions (Step 4). The image acquisition performed during the in situ sequencing can be performed using a wide field microscope (single focal plan) or using a confocal microscope (multiple focal plans).

Extraction of the cDNA containing the linear primer and UMI sequences from tissue (Step 5) and 2nd strand cDNA Synthesis using a branched primer adapter sequence (P2); (Step 6). PCR amplification of the cDNA with a branched adapter sequence complementary to the linear probe A region (P1) followed by P1/P2 adaptor primers (Step7) . Circularization of the PCR amplified product and RCA amplification of the circle which contains UMI and the region of interest (Step 8). NGS Sequencing of the region of interest and the UMI and determining the location and the sequences of the region of interest by matching the UMI sequence off the tissue and on the tissue with the corresponding coordinates (x.y and/or z); Step 9).

Figure 1 shows the first three steps of the process where a linear probe containing a random sequence flanked by two anchors (A & B) and a specific sequence complementary to the target region of interest mRNA gene which needs to be sequenced. The specific portion of the linear probe is hybridized to the target first followed by Reverse Transcription. The region of interest will contain a sequence suspected of nucleotide change of variant difference. Once the linear probe has been elongated creating a cDNA copy and is attached stably to the target, then the circle locator is hybridized to the linear probe via regions A & B, leaving a gap corresponding to the UMI region. The UMI region is copied by filling the gap and the circle locator is ligated, generating a circular molecule (Step2). The circular molecule is used as a template to generate DNA nanoballs (rolonies). The UMI portion of the rolonies are sequenced directly on tissue, providing the location coordinates of the target region (x.y and/or z).

Figure 1 shows further the hybridization of the linear probe containing a random sequence and two anchors (A & B) to mRNA on a fixed tissue on a slide to a region upstream from the region of interest in a particular mRNA (dotted line) via the specific sequence portion located at the 3'end. The reverse transcription of the region of interest onto a cDNA (Step 1) is initiated by the linear probe, that is used as a primer. The 3'end of the linear probe consists of sequence about 30 bp long which can hybridize to a specific region in an mRNA target of interest on tissue. Hybridization of the circle locator padlock molecule to the linear probe via regions A & B, leaving a gap corresponding to the UMI region is shown in step 2. The UMI region is copied to the circle locator padlock by filling the gap using a non strand-displacement enzyme for example Phusion DNA polymerase and ligation of the two extremities of the circle locator padlock using a DNA ligase, generating a circular molecule (step2). The size of the circle can be between 100 to 400bp long with the gap portion to be about 10-40bp .

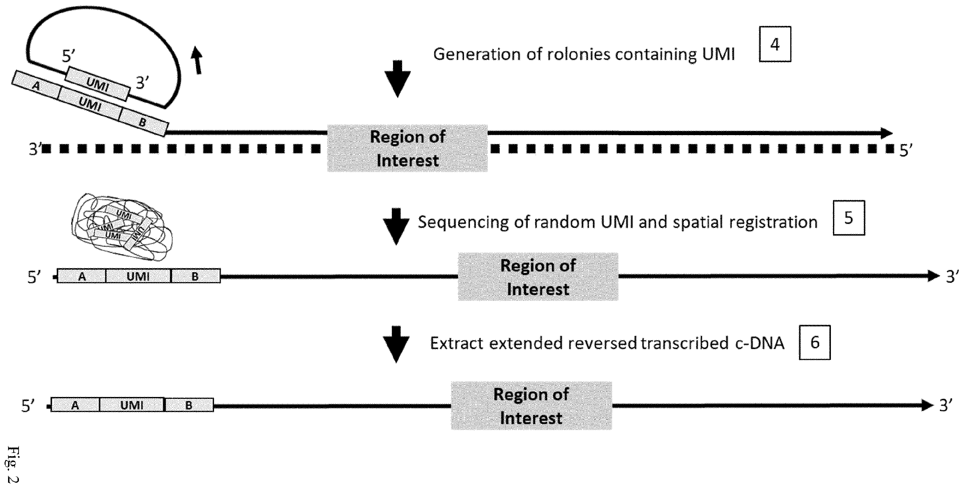

Figure 2 shows the circular molecule used as a template to generate DNA nanoballs. The circular molecule is amplified by (Rolling Circle Amplification) RCA (Step 3) and the UMI portion is sequenced in situ (Step 4). The cDNA strand containing a copied UMI is then extracted for sequencing of the region of interest (Step 5).

Figure 2 shows further the process of how the circle locator is used to generated DNA nanoballs (rolonies) on the tissue via isothermal rolling circle amplification (RCA) using an enzyme with highly processivity and strand displacement activity like Phi29 DNA polymerase and RCA oligonucleotide binding to circle locator as a primer (step 3). The resulting location of the RCA product on the tissue is determined by sequencing the randomly generated UMI on tissue and obtaining coordinates (x, y and/or z) of all the rolonies (step 4). In one embodiment of the invention, the circle locator probe, is hybridized first to the linear probe anchor before the latest binds to a region upstream from the region of interest in a particular mRNA (dotted line). The extended cDNA is extracted from the tissue section slide in step 5.

Figure 3 shows how the extracted cDNA strand from the tissue can be PCR amplified and later circularized by adding adaptor regions (P1 and P2) to the PCR primers. The 2^{nd} strand cDNA synthesis is performed in Step 6 and PCR amplified to generate double stranded DNA molecule with P1/P2 adaptor ends (Step 7) that can be analyzed by NGS sequencing (Step 8).

Figure 3 shows further the 2^{nd} strand of cDNA synthesis performed (step 6) and subsequent PCR reaction (step 7) performed using a pair of branched primers P1 and P2. The binding regions are located upstream of the circle locator region for Primer P1 and is located downstream of the "Region of Interest" for Primer P2 generating a adapted DNA library containing the region of interest and the UMI portion. Sequencing of the DNA library allows for determining determine if either a mutation/nucleotide variant exist is the target region of interest and by sequencing the UMI, to identify the original location/origin of each region of interest on the tissue by correlating them with the circle locator UMI sequences coordinates generated sequencing the rolonies on a tissue section (step 8).

### EXAMPLES

Sequencing of the DNA library allows to detect the presence of a mutation/nucleotide variant in the targeted region of interest, and by sequencing the UMI, to identify the original location/origin of each region of interest on the tissue by correlating them with the circle locator UMI sequences coordinates generated sequencing the rolonies on a tissue section (step 8).

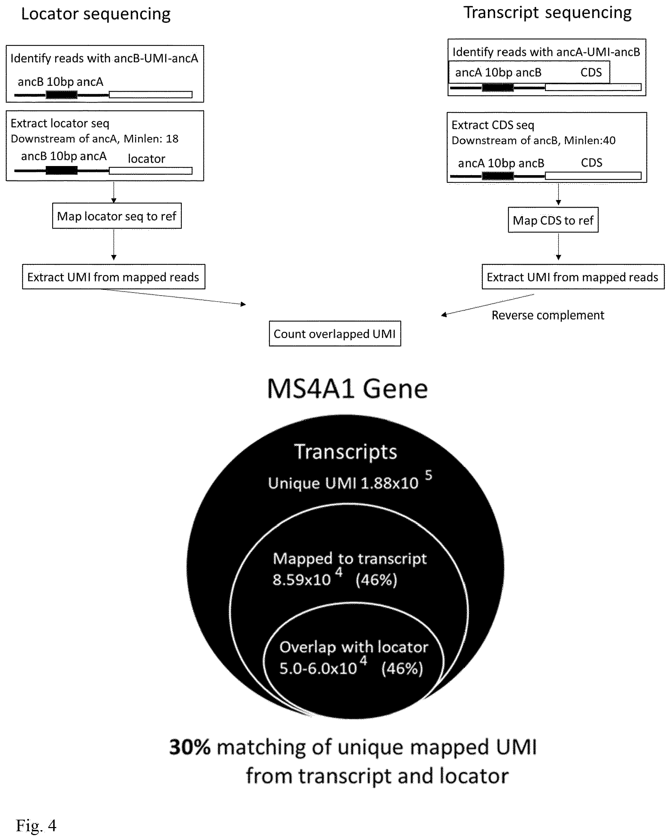

Figure 4 shows an example of the sequence analysis performed on libraries generated from extracted and PCR amplified cDNA (MS4A1 gene) and DNA nanoballs. The sequencing was performed ex-situ using a primer oligonucleotide located upstream of the region of interest and the unique molecular identifier (UMI) portions. The UMI portion along with the locator padlock is sequenced, and the reads originating from the locator rolonies were identified by mapping the locator sequence reads to the known padlock reference sequence. Additionally, the transcripts were sequenced also where the UMI portion as well as the coding sequence from the cDNA transcript containing the linear primer and UMI sequences that were copied to the locator padlock. The reads originating from the cDNA transcripts were identified by mapping the sequence reads to the known reference sequence of the coding region from the gene of interest (in this example MS4A1 gene).

Figure 5 shows the actual reads obtained from the libraries generated from extracted and PCR amplified cDNA the transcript portion and that actually mapped to the (MS4A1 gene). The sequencing was performed ex-situ using a primer oligonucleotide located upstream of the region of interest and the unique molecular identifier (UMI) portions. The coding sequence portion of MS4A1 that was generated by reverse transcription of the linear primer is highlighted (blocked region)

## Claims

1. A method to obtain the spatial location and sequence information of a target sequence of at least one m-RNA strand on a tissue sample comprising the steps
a. providing a linear probe, containing a) a binding region capable of binding to the at least one m-RNA strand and b) an anchor sequence comprising a UMI region located between a first and a second locator regions and c) a primer region;
b. hybridizing the linear probe with its binding region to the m-RNA strand;
c. complementing the linear probe using the m-RNA strand as template thereby obtaining a reversed transcribed c-DNA strand
d. hybridizing a locator molecule with its 3' and 5' ends to the first and second locator regions thereby creating a gap corresponding to the length of the UMI of the linear probe
e. Filling the gap in the locator molecule with nucleotides complementary to the UMI using a non-strand displacement enzyme thereby creating a circular template comprising a copy of the UMI region from the linear probe.
f. multiplying the circular template molecule by RCA on the tissue sample, starting from a primer region thereby creating a rolony
g. Sequencing at least the UMI portion of the rolonies thereby obtaining the spatial location of the m-RNA on the tissue
h. removing the reversed transcribed c-DNA strand from the tissue and dehybridizing the m-RNA strand thereby obtaining a single stranded c-DNA oligomer
i. providing the single stranded cDNA oligomer with a first and a second adaptor primer at the 3' and 5' ends obtaining a primed single stranded oligomer; amplification of the primed single stranded oligomer by PCR
j. Sequencing the amplified primed single stranded oligomer and linking the spatial information of the rolonies with the sequence information of the amplified primed single stranded oligomer via the UMI sequence

2. Method according to claim 1 **characterized in that** steps a) to d) are performed in the sequence a), b), c) and d).

3. Method according to claim 1 **characterized in that** steps a) to d) are performed in the sequence a), d), b), and c).

4. Method according to any of the claims 1 to 3 **characterized in that** filling the gap of the locator molecule in step e) is performed by Phusion DNA Polymerase or non-strand displacement polymerase using a primer hybridized to a region of the circular locator.

5. Method according to any of the claims 1 to 4 **characterized in that** the 3' and 5' ends of the gap-filled DNA molecule are ligated with each other wherein the UMI and the first region of the linear locator acts as a bridge splint.

6. Method according to claim 5 **characterized in that** the ligation reaction in step e) is performed by a DNA ligase

7. Method according to claim 6 **characterized in that** the locator probe is provided by first hybridizing the linear locator to the at least one m-RNA strand, complementing the RNA anchor region of the locator probe into a reversed transcribed c-DNA strand and then hybridizing the circular locator to the linear locator.

8. Method according to any of the claims 1 to 7 **characterized in that** the single stranded oligomer is physically sheared to smaller fragment before adding a first and a second adaptor primer at the 3' and 5' ends

9. Method according to any of the claims 1 to 8 **characterized in that** the single stranded adapted DNA oligomer is circularized and multiplied by RCA into second rolonies before sequencing

## Patentansprüche

1. Verfahren zum Gewinnen der räumlichen Lage und Sequenzinformation einer Zielsequenz von mindestens einem m-RNA-Strang auf einer Gewebeprobe, umfassend die folgenden Schritte:
a. Bereitstellen einer linearen Sonde, enthaltend a) eine Bindungsregion, die in der Lage ist, an den mindestens einen m-RNA-Strang zu binden, und b) eine Ankersequenz, umfassend eine UMI-Region, die sich zwischen einer ersten und einer zweiten Locator-Region befindet, und c) eine Primer-Region;
b. Hybridisieren der linearen Sonde mit ihrer Bindungsregion an den m-RNA-Strang;
c. Komplementieren der linearen Sonde unter Verwendung des m-RNA-Strangs als Matrize, wodurch ein revers transkribierter c-DNA-Strang erhalten wird;
d. Hybridisieren eines Locator-Moleküls mit seinem 3'- und 5'-Ende an die erste und zweite Locator-Region, wodurch eine Lücke erzeugt wird, die der Länge des UMI der linearen Sonde entspricht;
e. Füllen der Lücke im Locator-Molekül mit Nukleotiden, die zum UMI komplementär sind, unter Verwendung eines Nicht-Strand-Displacement-Enzyms, wodurch eine zirkuläre Matrize entsteht, die eine Kopie der UMI-Region der linearen Sonde umfasst;
f. Vervielfältigen des zirkulären Matrizenmoleküls durch RCA auf der Gewebeprobe, ausgehend von einer Primer-Region, wodurch eine Rolonie entsteht;
g. Sequenzieren mindestens des UMI-Abschnitts der Rolonies, wodurch die räumliche Lage der m-RNA auf dem Gewebe ermittelt wird;
h. Entfernen des revers transkribierten c-DNA-Strangs aus dem Gewebe und Dehybridisieren des m-RNA-Strangs, wodurch ein einzelsträngiges c-DNA-Oligomer erhalten wird;
i. Bereitstellen des einzelsträngigen c-DNA-Oligomers mit einem ersten und einem zweiten Adaptor-Primer an den 3'- und 5'-Enden, wodurch ein geprimtes einzelsträngiges Oligomer erhalten wird; Amplifizieren des geprimten einzelsträngigen Oligomers durch PCR;
j. Sequenzieren des amplifizierten geprimten einzelsträngigen Oligomers und Verknüpfen der räumlichen Informationen der Rolonies mit den Sequenzinformationen des amplifizierten geprimten einzelsträngigen Oligomers über die UMI-Sequenz.

2. Verfahren nach Anspruch 1, **dadurch gekennzeichnet, dass** die Schritte a) bis d) in der Reihenfolge a), b), c) und d) durchgeführt werden.

3. Verfahren nach Anspruch 1, **dadurch gekennzeichnet, dass** die Schritte a) bis d) in der Reihenfolge a), d), b) und c) durchgeführt werden.

4. Verfahren nach einem der Ansprüche 1 bis 3, **dadurch gekennzeichnet, dass** das Füllen der Lücke des Locator-Moleküls in Schritt e) durch Phusions-DNA-Polymerase oder Nicht-Strand-Displacement-Polymerase unter Verwendung eines Primers, der an eine Region des zirkulären Locators hybridisiert ist, durchgeführt wird.

5. Verfahren nach einem der Ansprüche 1 bis 4, **dadurch gekennzeichnet, dass** die 3'- und 5'-Enden des lückengefüllten DNA-Moleküls miteinander ligiert werden, wobei der UMI und die erste Region des linearen Locators als Brücken-Splint wirken.

6. Verfahren nach Anspruch 5, **dadurch gekennzeichnet, dass** die Ligationsreaktion in Schritt e) durch eine DNA-Ligase durchgeführt wird.

7. Verfahren nach Anspruch 6, **dadurch gekennzeichnet, dass** die Locator-Sonde bereitgestellt wird, indem zuerst der lineare Locator an den mindestens einen m-RNA-Strang hybridisiert wird, die RNA-Ankerregion der Locator-Sonde zu einem revers transkribierten c-DNA-Strang komplementiert wird und dann der zirkuläre Locator an den linearen Locator hybridisiert wird.

8. Verfahren nach einem der Ansprüche 1 bis 7, **dadurch gekennzeichnet, dass** das einzelsträngige Oligomer physikalisch zu einem kleineren Fragment geschert wird, bevor ein erster und ein zweiter Adaptor-Primer an den 3'- und 5'-Enden hinzugefügt werden.

9. Verfahren nach einem der Ansprüche 1 bis 8, **dadurch gekennzeichnet, dass** das einzelsträngige adaptierte DNA-Oligomer vor der Sequenzierung zirkularisiert und durch RCA in zweite Kolonien vervielfältigt wird.

## Revendications

1. Procédé d'obtention de la localisation spatiale et des informations de séquence d'une séquence cible d'au moins un brin d'ARNm sur un échantillon de tissu comprenant les étapes consistant à
a. fournir une sonde linéaire, contenant a) une région de liaison capable de se lier à au moins un brin d'ARNm et b) une séquence ancre comprenant une région UMI située entre une première et une seconde régions locatrices et c) une région d'amorce ;
b. hybrider la sonde linéaire avec sa région de liaison au brin d'ARNm ;
c. complémenter la sonde linéaire en utilisant le brin d'ARNm comme matrice obtenant ainsi un brin d' ADNc transcrit en inverse
d. hybrider une molécule locatrice avec ses extrémités 3' et 5' à la première et seconde régions locatrices créant ainsi un espace libre correspondant à la longueur de l'UMI de la sonde linéaire
e. remplir l'espace libre dans la molécule locatrice avec des nucléotides complémentaires à l'UMI en utilisant une enzyme de déplacement de non brin créant ainsi une matrice circulaire comprenant une copie de la région UMI de la sonde linéaire
f. multiplier la molécule de matrice circulaire par RCA sur l'échantillon de tissu, commençant à partir d'une région d'amorce créant ainsi une rolonie
g. séquencer au moins la portion UMI des rolonies obtenant ainsi la localisation spatiale de l'ARNm sur le tissu
h. enlever le brin d'ADNc transcrit en inverse du tissu et déshybrider le brin d'ARNm obtenant ainsi un oligomère d'ADNc simple brin
i. fournir à l'oligomère d'ADNc simple brin une première et une seconde amorces adaptatrices aux extrémités 3' et 5' obtenant un oligomère simple brin amorcé ; l'amplification de l'ADNc oligomère simple brin amorcé par PCR
j. séquencer l'oligomère simple brin amorcé amplifié et lier les informations spatiales des rolonies avec les informations de séquence de l'oligomère simple brin amorcé amplifié via la séquence UMI.

2. Procédé selon la revendication 1 **caractérisé en ce que** les étapes a) à d) sont effectuées dans la séquence a), b), c) et d).

3. Procédé selon la revendication 1 **caractérisé en ce que** les étapes a) à d) sont effectuées dans la séquence a), d), b) et c).

4. Procédé selon l'une quelconque des revendications 1 à 3 **caractérisé en ce que** le remplissage de l'espace libre de la molécule locatrice dans l'étape e) est effectué par une ADN polymérase Phusion ou une polymérase de déplacement de non brin en utilisant une amorce hybridée à une région du locateur circulaire.

5. Procédé selon l'une quelconque des revendications 1 à 4 **caractérisé en ce que** les extrémités 3' et 5' de la molécule d'ADN à espace libre rempli sont liguées l'une avec l'autre l'UMI et la première région du locateur linéaire agissant comme une attelle de pont.

6. Procédé selon la revendication 5 **caractérisé en ce que** la réaction de ligation dans l'étape e) est effectuée par une ligase d'ADN.

7. Procédé selon la revendication 6 **caractérisé en ce que** la sonde locatrice est fournie par la première hybridation du locateur linéaire à l'au moins un brin d'ARNm, complémentant la région ancre d'ARN de la sonde locatrice dans un brin d'ADNc transcrit en inverse puis en hybridant le locateur circulaire au locateur linéaire.

8. Procédé selon l'une quelconque des revendications 1 à 7 **caractérisé en ce que** l'oligomère simple brin est physiquement découpé en un fragment plus petit avant d'ajouter une première et une seconde amorces adaptatrices aux extrémités 3' et 5'.

9. Procédé selon l'une quelconque des revendications 1 à 8 **caractérisé en ce que** l'oligomère d'ADN adapté simple brin est circularisé et multiplié par RCA en secondes rolonies avant le séquençage.
